# EUROPEAN PATENT SPECIFICATION

(11) **EP 3 768 186 B1**
(45) Date of publication and mention of the grant of the patent: **14.09.2022**
(21) Application number: 19734188.6
(22) Date of filing: 30.05.2019
(51) Int. Cl.: A61B 18/14, H05K 1/00, H05K 1/02, H05K 3/46

(54) **CATHETER HANDLE WITH COMPLIANT CIRCUIT**
KATHETERHANDGRIFF MIT NACHGIEBIGER SCHALTUNG
POIGNÉE DE CATHÉTER AVEC CIRCUIT SOUPLE

(30) Priority: 31.05.2018 US 201862678763 P
(43) Date of publication of application: 27.01.2021
(73) Proprietor: St. Jude Medical, Cardiology Division, Inc., St. Paul, MN 55117 (US)
(72) Inventor: OLSON, Gregory K., Elk River, Minnesota 55122 (US)
(74) Representative: Kramer Barske Schmidtchen Patentanwälte PartG mbB
(86) International application number: PCT/US2019/034725
(87) International publication number: WO 2019/232256

(56) References cited:
- WO-A1-2011/112931
- WO-A1-2012/124391
- US-A- 5 499 981
- US-A1- 2003 233 087
- US-A1- 2007 173 814
- US-B2- 9 171 794
- US-B2- 9 642 264

## Description

### BACKGROUND

### a. Field

The instant disclosure relates to catheters, such as catheter handles. In one example, the instant disclosure relates to catheter handles including a compliant circuit.

### b. Background Art

For many years, catheters have been used for cardiac medical procedures. For example, catheters can be used to diagnose and treat cardiac arrhythmias. Catheters can be positioned at a specific location within a body that is otherwise inaccessible without a more invasive procedure. Often, catheters can be in communication with a control unit for receiving signals from electrodes or sensors of the catheter. The signals can be communicated through one or more insulated wires. The insulated wires can be routed through a handle of the catheter. The wires can be terminated with a connector or soldered to complete electrical connection between the electrodes, sensors, and the control unit. The size and shape of the handle can be configured to fit into the hand of an operator and to provide proper control of the catheter by the operator.

An exemplary electrode catheter is known from WO 2012/124391 A1. Another medical device is known from US 2007/0173814 A1. A medical or dental handle is known from US 9,642,264 B2.

In many instances, catheters are used in surgical operating rooms. Surgical operating rooms can be an electrically noisy environment. For instance, the electronic components within the surgical operating rooms can be exposed to electro-magnetic interference from other devices within the surgical operating room. In an example, an electric-field-based positioning system can generate an electronic field for identifying a position of a medical device, such as the catheter, within a patient's body. The electronic field can contribute to the electro-magnetic interference.

The foregoing discussion is intended only to illustrate the present field and should not be taken as a disavowal of claim scope.

### BRIEF SUMMARY

The invention is as defined in independent claims 1 and 10. Dependent claims relate to exemplary embodiments.

The instant disclosure relates to a catheter, such as a high-density mapping catheter, a mapping catheter for diagnosis, or a catheter for medical treatment. For example, the catheter can be used for the treatment of cardiac arrhythmias, for instance, via ablation. Some examples of the catheter can include irrigation. In particular, the instant disclosure relates to a handle of a catheter, such as a handle including a compliant circuit configured to be disposed on a non-planar surface of the handle. As used herein, the term compliant layer or circuit should be understood to mean a layer or circuit that conforms closely to the shape of an adjacent proximate surface, including a non-planar surface.

In an example, a catheter can include a catheter shaft, an electrical conductor, a catheter handle, and a compliant circuit. The catheter shaft can include a proximal end and a distal end having at least one electrode or sensor. The electrical conductor can be coupled to the catheter shaft. The electrical conductor can be communicatively coupled to the electrode or the sensor located at the distal end of the catheter shaft. The handle can be coupled to the proximal end of the catheter shaft and can include a non-planar surface. The compliant circuit can be configured for communication with an electronic control unit and to conform to the non-planar surface. In an example, the compliant circuit can be electrically coupled to the electrode or the sensor through the electrical conductor. In various examples, the compliant circuit can include a material characteristic to stretch and comply with the non-planar surface. In further examples, the compliant circuit can include at least one dielectric layer and at least one conductive layer. At least one of the dielectric layers and at least one of the conductive layers can include a respective material characteristic. In an example, the conductive layer can include a printable conductive polymer, such as a conductive ink.

In some examples, the compliant circuit can include a logic component embedded within the compliant circuit. The logic component can be electrically coupled to at least one of the conductive layers. In various examples, the logic component can be configured as one of an analog-to-digital converter, a multiplexer, a filter, an amplifier, or a combination thereof. In an example, the logic component can include a semiconductor. For instance, the logic component can include a p-type material or an n-type material.

In an example, a method of making a catheter handle can include disposing a compliant circuit along a non-planar surface of the catheter handle. The method can further include conforming the compliant circuit to the non-planar surface. In some examples, the compliant circuit includes a dielectric layer, a plurality of conductive layers, a first connection interface, and a second connection interface. The dielectric layer can include a dielectric material characteristic, and each of the plurality of conductive layers can include a conductive material characteristic. The first connection interface can be electrically coupled to at least one of the conductive layers. The second connection interface can be electrically coupled to at least one of the conductive layers. In some instance, the at least one conductive layer can include a strain relief feature. In an example, at least one of the conductive layers can be a printed conductive layer, such as a printed conductive polymer, conductive ink, or the like. In further examples, a logic component can be formed within the compliant circuit. The logic component can include a semiconductor. For instance, the logic component can include a p-type material, an n-type material, or both.

The foregoing and other aspects, features, details, utilities, and advantages of the present disclosure will be apparent from reading the following description and claims, and from reviewing the accompanying drawings.

### BRIEF DESCRIPTION OF THE DRAWINGS

**FIG. 1** is an example of a system for navigating a medical device within a body, the system including a catheter, according to the present description.
**FIG. 2** is an example of a compliant circuit, according to the present description.
**FIG. 3** is an example of an exploded view of a handle of a catheter having a compliant circuit disposed on a non-planar surface of the handle, according to the present description.
**FIG. 4** is an example of a cross section of a compliant circuit including at least one logic component, according to the present description.
**FIG. 5** is an example of a method for making a catheter assembly including a compliant circuit disposed on a handle, according to the present description.

### DETAILED DESCRIPTION OF EMBODIMENTS

The instant disclosure relates to a catheter, such as a high-density mapping catheter, a mapping catheter for diagnosis, or a catheter for medical treatment. For example, the catheter can be used for the treatment of cardiac arrhythmias, for instance, via ablation. In some examples, the catheter of the present disclosure can include an irrigated catheter configured to irrigate tissue during an ablation procedure. In particular, the instant disclosure relates to a handle of a catheter, such as a handle including a compliant circuit configured to be disposed on a surface, such as a non-planar surface, of the handle. Details of the various examples of the present disclosure are described below with specific reference to the figures.

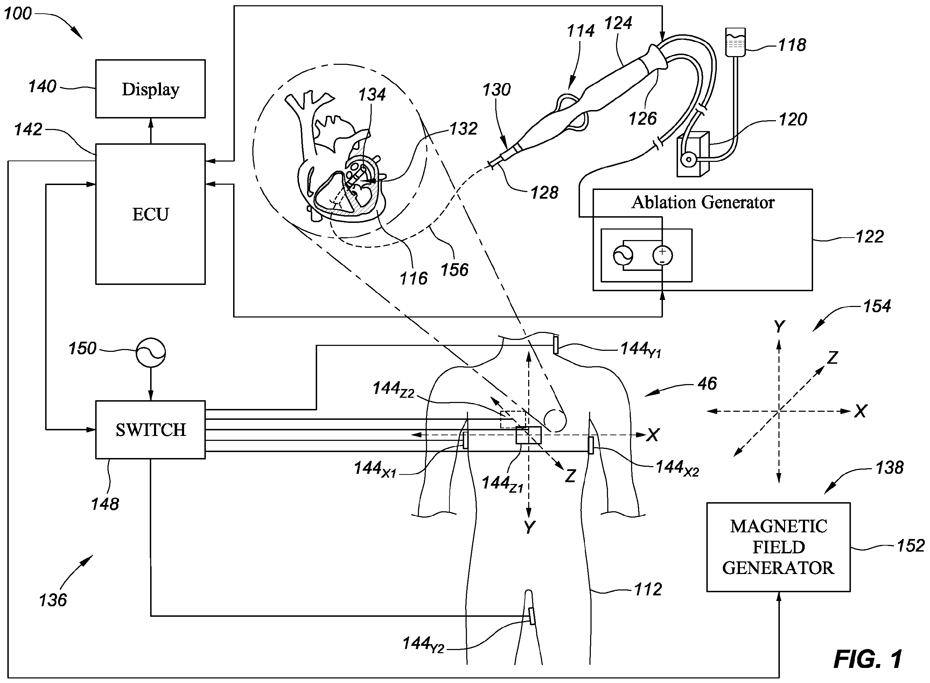

**FIG. 1** illustrates one example of a system 100 for navigating a medical device within a body 112. In the illustrated example, the medical device comprises a catheter 114 that is shown schematically entering a heart that has been exploded away from the body 112. The catheter 114, in this example, is depicted as an irrigated radiofrequency (RF) ablation catheter for use in the treatment of cardiac tissue 116 in the body 112. It should be understood, however, that the system 100 can find application in connection with a wide variety of medical devices used within the body 112 for diagnosis or treatment. For example, the system 100 can be used to navigate an electrophysiological mapping catheter, an intracardiac echocardiography (ICE) catheter, or an ablation catheter using a different type of ablation energy (e.g., cryoablation, ultrasound, etc.). Further, it should be understood that the system 100 can be used to navigate medical devices used in the diagnosis or treatment of portions of the body 112 other than cardiac tissue 116. Further description of the systems and components are contained in U.S. patent application 13/839,963 filed on 15 March 2013, published as US 2014-0275991 A1.

Referring still to FIG. 1, the ablation catheter 114 is connected to a fluid source 118 for delivering a biocompatible irrigation fluid such as saline through a pump 120, which can comprise, for example, a fixed rate roller pump or variable volume syringe pump with a gravity feed supply from fluid source 118 as shown. The catheter 114 is also electrically connected to an ablation generator 122 for delivery of RF energy. The catheter 114 can include a handle 124, a cable connector or interface 126 at a proximal end of the handle 124, and a shaft 128. The shaft 128 can include a proximal end 130, a distal end 132, and one or more electrodes 134. The connector 126 provides mechanical, fluid, and electrical connections for conduits or cables extending from the pump 120 and the ablation generator 122. The catheter 114 can also include other conventional components not illustrated herein such as a temperature sensor, additional electrodes, and corresponding conductors or leads.

The handle 124 provides a location for the physician to hold the catheter 114 and can further provide means for steering or guiding the shaft 128 within the body 112. For example, the handle 124 can include means to change the length of one or more pull wires extending through the catheter 114 from the handle 124 to the distal end 132 of shaft 128. The construction of the handle 124 can vary. In various examples, the handle 124 can be configured to fit within a user's hand, for instance, to provide control over the operation of the catheter 114. Accordingly, the size and shape of the handle 124 can be important for the function and usability of the catheter 114.

The shaft 128 can be made from conventional materials such as polyurethane and can define one or more lumens configured to house and/or transport electrical conductors 156, fluids, or surgical tools. The shaft 128 can be introduced into a blood vessel or other structure within the body 112 through a conventional introducer. The shaft 128 can then be steered or guided through the body 112 to a desired location such as the tissue 116 using guide wires or pull wires or other means known in the art including remote control guidance systems. The shaft 128 can also permit transport, delivery, and/or removal of fluids (including irrigation fluids and bodily fluids), medicines, and/or surgical tools or instruments. It should be noted that any number of methods can be used to introduce the shaft 128 to areas within the body 112. This can include introducers, sheaths, guide sheaths, guide members, guide wires, or other similar devices. For ease of discussion, the term introducer will be used throughout.

The system 100 can include an electric-field-based positioning system 136, a magnetic-field-based positioning system 138, a display 140, and an electronic control unit (ECU) 142 (e.g., a processor). Each of the exemplary system components is described further below.

The electric-field-based positioning system 136 and the magnetic-field-based positioning system 138 are provided to determine the position and orientation of the catheter 114 and similar devices within the body 112. The position and orientation of the catheter 114 and similar devices within the body 112 can be determined by the system 136 and/or the system 138. The system 136 can comprise, for example, the EnSite^{™} NavX^{™} system sold by St. Jude Medical, Inc. of St. Paul, Minnesota, and described in, for example, U.S. Patent No. 7,263,397 titled "Method and Apparatus for Catheter Navigation and Location Mapping in the Heart,". The systems 136 and 138 can comprise, for example, the EnSite Precision^{™} system sold by St. Jude Medical, Inc., of St. Paul, Minnesota. The system 136 operates based upon the principle that when low amplitude electrical signals are passed through the thorax, the body 112 acts as a voltage divider (or potentiometer or rheostat) such that the electrical potential or field strength measured at one or more electrodes 134 on the catheter 114 can be used to determine the position of the electrodes, and, therefore, of the catheter 114, relative to a pair of external patch electrodes using Ohm's law and the relative location of a reference electrode (e.g., in the coronary sinus).

In the configuration shown in FIG. 1, the electric-field-based positioning system 136 further includes three pairs of patch electrodes 144, which are provided to generate electrical signals used in determining the position of the catheter 114 within a three-dimensional coordinate system 146. The electrodes 144 can also be used to generate electrophysiology (EP) data regarding the tissue 116. To create axes-specific electric fields within body 112, the patch electrodes are placed on opposed surfaces of the body 112 (e.g., chest and back, left and right sides of the thorax, and neck and leg) and form generally orthogonal X, Y, and Z axes. A reference electrode/patch (not shown) is typically placed near the stomach and provides a reference value and acts as the origin of the coordinate system 146 for the navigation system.

In accordance with this exemplary system 136 as depicted in FIG. 1, the patch electrodes include right side patch 144xi, left side patch 144_{X2}, neck patch 144_{Y1}, leg patch 144_{Y2}, chest patch 144zi, and back patch 144_{Z2}; and each patch electrode is connected to a switch 148 (e.g., a multiplex switch) and a signal generator 150. The patch electrodes 144_{X1}, 144_{X2} are placed along a first (X) axis; the patch electrodes 144_{Y1}, 144_{Y2} are placed along a second (Y) axis, and the patch electrodes 144zi, 144_{Z2} are placed along a third (Z) axis. Sinusoidal currents are driven through each pair of patch electrodes, and voltage measurements for one or more position sensors (e.g., ring electrodes 134 or a tip electrode located near the distal end 132 of catheter shaft 128) associated with the catheter 114 are obtained. The measured voltages are a function of the distance of the position sensors from the patch electrodes. The measured voltages are compared to the potential at the reference electrode and a position of the position sensors within the coordinate system 146 of the navigation system is determined.

The magnetic-field-based positioning system 138 in this example employs magnetic fields to detect the position and orientation of the catheter 114 within the body 112. The system 138 can include the GMPS system made available by MediGuide, Ltd. and generally shown and described in, for example, U.S. Patent No. 7,386,339 titled "Medical Imaging and Navigation System,". In such a system, a magnetic field generator 152 can be employed having three orthogonally arranged coils (not shown) to create a magnetic field within the body 112 and to control the strength, orientation, and frequency of the field. The magnetic field generator 152 can be located above or below the patient (e.g., under a patient table) or in another appropriate location. Magnetic fields are generated by the coils and current or voltage measurements for one or more position sensors (not shown) associated with the catheter 114 are obtained. The measured currents or voltages are proportional to the distance of the sensors from the coils, thereby allowing determination of a position of the sensors within a coordinate system 154 of system 138.

The display 140 is provided to convey information to a physician to assist in diagnosis and treatment. The display 140 can comprise one or more conventional computer monitors or other display devices. The display 140 can present a graphical user interface (GUI) to the physician. The GUI can include a variety of information including, for example, an image of the geometry of the tissue 116, electrophysiology data associated with the tissue 116, graphs illustrating voltage levels over time for various electrodes 134, and images of the catheter 114 and other medical devices and related information indicative of the position of the catheter 114 and other devices relative to the tissue 116.

The ECU 142 provides a means for controlling the operation of various components of the system 100, including the catheter 114, the ablation generator 122, and magnetic generator 152 of the magnetic-field-based positioning system 138. The ECU 142 can also provide a means for determining the geometry of the tissue 116, electrophysiology characteristics of the tissue 116, and the position and orientation of the catheter 114 relative to tissue 116 and the body 112. The ECU 142 also provides a means for generating display signals used to control the display 140.

As the catheter 114 moves within the body 112, and within the electric field generated by the electric-field-based positioning system 136, the voltage readings from the electrodes 134 change, thereby indicating the location of catheter 114 within the electric field and within the coordinate system 146 established by the system 136. The ring electrodes 134 can be adapted to communicate position signals to the ECU 142.

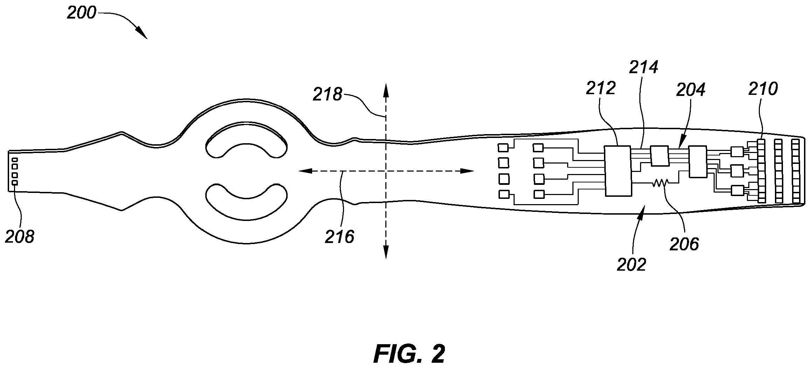

**FIG. 2** is, according to the invention, a flexible circuit, such as a compliant circuit 200. The compliant circuit 200 routes signals between the ECU 142 and an electrode or sensor of the shaft 128. For instance, the signals can include electrophysiological signals. The compliant circuit 200 can include a dielectric layer 202, a conductive layer 204, a first connection interface 208, and a second conductive interface 210. The compliant circuit 200 can include a plurality of routing traces configured to communicate signals between the ECU 142 and the electrodes 134 or sensors of the shaft 128. One or more conductive layers 204 can be disposed on one or more of the dielectric layers 202 to communicate the signals within the compliant circuit 200. For instance, the conductive layer 204 can be configured to include the plurality of routing traces 214 to communicate various signals within the compliant circuit 200. Often, catheters use insulated wires for communicating electrical signals between the ECU 142 and the various electrodes 134 or sensor. The traces 214 can include a trace thickness, width, spacing, or combination thereof that provides for dense circuit routing. For instance, the plurality of traces 214 of the compliant circuit 200 can have a higher density than insulated wires. In an example, the width of the trace 214 can be 0.01 to 0.25 mm and the spacing of the trace 214 can be 0.01 to 0.25 mm. Other trace thicknesses, widths, and spacing are also contemplated. Various conductive layers 204 can be electrically coupled by one or more conductive vias. In some instances, the size of the handle 124 can limit the number of insulated wires that can be disposed therein for communicating signals with the various electrodes 134 and sensors. Due to the relatively high density of the traces 214, the compliant circuit 200 can have a relatively small size as compared to a corresponding number of insulated wires. Accordingly, compared to insulated wires, the compliant circuit 200 can communicate a greater number of signal traces 214 through the limited size and shape of the handle 124. For instance, the compliant circuit 200 can communicate signals from a plurality of electrical conductors 156, such as 10, 50, 100, 200, 250, or other number of signals corresponding to the number of electrical conductors 156.

In some examples, the dielectric layer 202 and the conductive layer 204 can be compliant. For instance, the conductive layer 204 can be compliant (also referred to herein as a compliant conductive layer), and the dielectric layer 202 can be compliant (also referred to herein as a compliant dielectric layer). The compliant conductive layer 204 and the compliant dielectric layer 202 can be used independently or in combination with less compliant materials, such as copper foil, polyimide, FR4, or the like.

In various examples, a material of the compliant dielectric layer (dielectric material) can include, but is not limited to, silicone, polydimethylsiloxane (PDMS), polyethylene terephthalate (PET), polyurethane [e.g., thermal plastic polyurethane (TPU)], poly(p-xylylene) polymer, poly(methyl-methacrylate)(PMMA), poly[styrene-b-(ethylene-co-butylene)-b-styrene] triblock copolymer (SEBS), ionic liquid [1-butyl-3-methylimidazolium bis (trifluoromethanesulfonyl)imide (BMITFSI)], fluorinated copolymer, fluorinated rubber, silicone, cellulose fibers, polyester fibers, poly(styrene-co-ethylenebutylene-co-styrene), graphene, polyacrimide hydrogel with Sodium chloride (NaCl) electrolyte, poly(2-methacrylolyloxyethyltrimethylammonium chloride)(PMETAC), silicon dioxide (SiO₂), poly(tert-butylacrylate-co-acrylic acid) poly(TBA-co-AA) film (a bistable electroactive polymer), Polytetrafluoroethylene (PTFE), or the like.

In some examples, a material of the compliant conductive layer (conductive material) can include, but is not limited to, a conductive stretchable polymer, a printable conductive polymer, a conductive stretchable ink, indium tin oxide (ITO), single-wall carbon nanotubes, multi-wall carbon nanotubes, carbon nanotube ribbons, carbon nanotube aerogel, poly[styrene-b- (ethylene-co-butylene)-b-styrene] triblock copolymer compounded with single-wall or multi-wall carbon nanotubes, Polyaniline (PANI) covalently bonded to poly(styrene-co-ethylenebutylene-co-styrene, Poly(3,4-ethylenedioxythiophene) :poly(styrenesulfonate)(PEDOT:PSS), poly(3,4-ethylenediox-ythiophene):p-tosylate (PEDOT), polyacrylic acid (PAA) implanted gold (Au) or titanium (Ti) or palladium (Pd) ions, copper (e.g., copper nanoparticles), gold (e.g., gold nanoparticles), silver (e.g., silver nanoparticles), zinc (e.g., zinc nanoparticles), spherical citrate stabilized Au nanoparticles, Au nanowires, gallium arsenide (GaAs) nanoribbons, silicon (Si) nanoribbons, silicon nanowires, graphene and silver nanowire hybrid foam, or the like. Where compliant conductive layer includes a rigid material, such as gold, titanium, palladium, copper, silver, zinc, or the like, the conductive material can be combined with a more compliant material to form a composite material. In another example, the conductive material can include at least one strain relief feature 206 (as described below) to increase the extensibility and compliance of the conductive material to form the compliant conductive material.

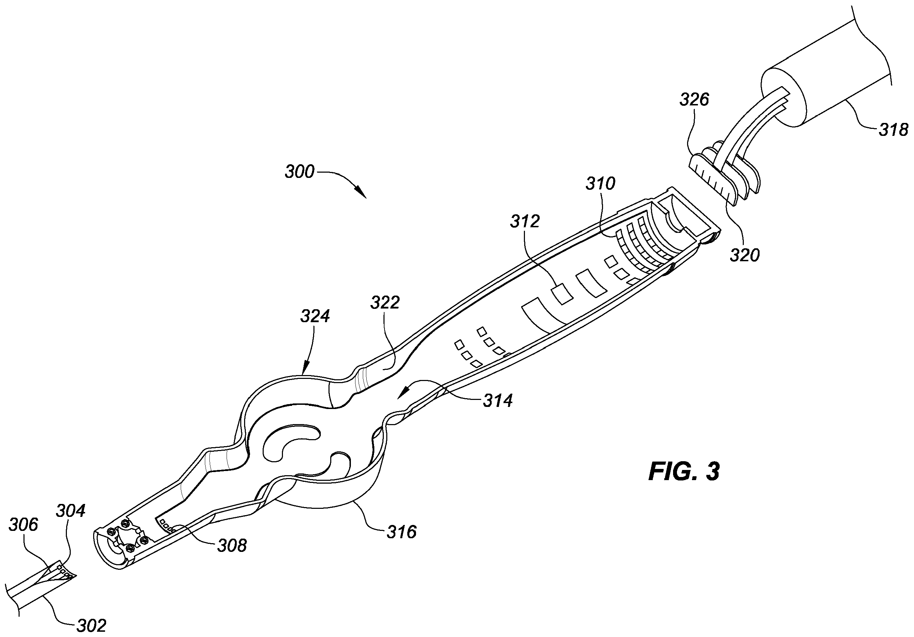

The dielectric layer 202, the conductive layer 204, or both can each include a respective material characteristic (e.g., elasticity characteristic), such as a dielectric material characteristic and a conductive material characteristic. For instance, the compliant circuit 200 can be conformable along a length and a width of the compliant circuit 200 based on the respective dielectric material and conductive material characteristics of the various layers (e.g., dielectric layer 202 and conductive layer 204), other elements of the compliant circuit 200, and the compliant circuit 200 as a whole. In an example, the compliant circuit 200 can bend or stretch along a first axis (e.g., X-axis 216), a second axis (e.g., Y-axis 218), or any combination thereof simultaneously based on the respective materials characteristics of the compliant circuit 200. For instance, the compliant circuit can stretch or bend along the first and second axes simultaneously while conforming to a non-planar surface. In some examples, the conductive material characteristic, the dielectric material characteristic, or both can include an elastic modulus that is less than that of steel, aluminum, ceramic, glass, polycarbonate, polyimide, or the like. For instance, the elastic modulus of the material can be 0.1 GPa to 2.0 GPa, 0.1 GPa to 1.5 GPa, 0.1 GPa to 1.0 GPa, or 0.1 GPa to 0.5 GPa. In a further example, the material, such as the dielectric material or the conductive material can permanently deform when stretched or conformed to the non-planar surface based on the respective material characteristics. For instance, when stretched or conformed, the dielectric material or the conductive material, in some examples, will not return to an original shape. Accordingly, the compliant circuit 200 can be disposed along a non-planar surface. The compliant circuit 200 can conform to the non-planar surface as shown in FIG. 3 and described herein. In an example, the compliant circuit 200 can take a shape that corresponds to a shape of the non-planar surface. Stated another way, the compliant circuit 200 can be conformable to the non-planar surface. The non-planar surface can include any non-planar shape, such as an arcuate shape, angular shape, irregular shape, or other non-planar shape.

In an example, the conductive layer 204 can include a strain relief feature 206. The strain relief feature 206 can increase the flexibility of the one or more traces, such as trace 214, of the conductive layer 204. The strain relief feature 206 can include, but is not limited to, a zigzag, horseshoe, wave, open loop, or other geometry that can mitigate failure of the conductive layer 204 when under strain. For instance, the strain relief feature 206 can increase the extensible length of the trace 214. In some examples, the conductive layer 204 can be a metallic layer, such as copper or a copper alloy. The strain relief feature 206 can provide compliant properties to the conductive layer 204 so the conductive layer 204 can be conformable. For instance, the material characteristic of the conductive layer 204 can be based, at least in part, on the strain relief feature 206.

As shown in the example of FIG. 2, the compliant circuit 200 includes at least one connection interface, such as a first connection interface 208 on the distal end of the compliant circuit 200 and a second connection interface 210 on the proximal end of the compliant circuit 200. The first connection interface 208 or the second connection interface 210 can include, but are not limited to, a solder pad, electrical connector, spring, probe contact, or the like. In the example of FIG. 2, the first connection interface 208 and the second connection interface 210 are solder pads. The first connection interface 208 can provide an electrical connection to one or more of the electrical conductors 156 of the shaft 128. The second connection interface 210 can provide an electrical connection to the ECU 142 or the ablation generator 122. Accordingly, the compliant circuit 200 can be electrically coupled to the one or more electrodes 132 or sensors (e.g., by virtue of one or more electrical conductors 156) and the ECU 142, ablation generator 122, or any combination thereof.

As further depicted in FIG. 2, the compliant circuit 200 can include at least one logic component 212. For instance, the logic component 212 can be included in or configured as a logic circuit or integrated circuit. In various examples, the logic component 212 can include, but is not limited to, an amplifier, multiplex circuit, transistor, diode, switch, analog-to-digital converter, isolator (filter), memory, or other logic element. The electric field generated by the electric-field-based positioning system 136 or the magnetic field generated by the magnetic-field-based positioning system 138 can interfere with one or more of the signals communicated through the electrical conductors 156. The logic component 212 can condition the signal to mitigate interference from electro-magnetic radiation. For instance, conditioning the signal can include, but is not limited to, reducing interference, boosting a signal-to-noise ratio, amplifying signal power, filtering the signal, or providing other signal adjustment. The signal-to-noise ratio or the strength of the signal received at the ECU 142 can be increased by the logic component 212. For instance, locating the logic component 212 in the handle 124 can reduce the length of the one or more electrical conductors 156 exposed to the interference. As a result, signals received at the handle 124 can have less interference than a corresponding signal communicated all the way to the ECU 142. Accordingly, applying signal conditioning using the logic component 212 can be more effective at the handle 124 than at the ECU 142. Thus, the integrity of the signal can be increased by locating the logic component 212 within the handle 124.

In another example, the logic component 212 can convert the signal between analog and digital. In some instances, digital signals can be less sensitive to interferences than analog signals. For instance, interference is often analog. The analog interference can be filtered from the digital signal to reduce the signal-to-noise ratio.

In a further example, the logic component 212 can multiplex the signal to reduce the number of electrical conductors for communicating with the ECU 142. Accordingly, the size of the compliant circuit can be reduced. Correspondingly, the size of the handle 124 can be reduced, for instance to provide an ergonomic shape.

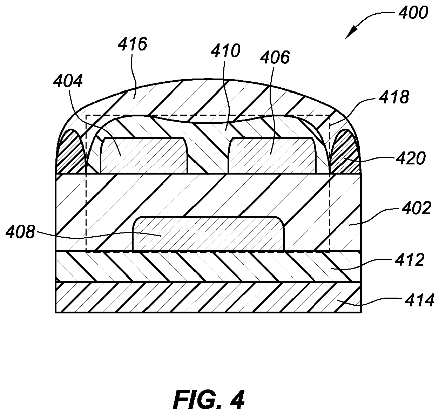

**FIG. 3** illustrates an exploded view of an example of a catheter 300 including a portion 316 of a handle 324, a compliant circuit 314, a shaft 302, and an electrical cable 318. According to the invention, the handle 324 includes a non-planar surface, such as non-planar surface 322. At least a portion of an interior of the handle 324 includes a non-planar surface 322. In some examples, the catheter handle can include a combination of planar and non-planar surfaces. In another example, the catheter handle can have planar side surfaces and a curved edge joining the side surface with an adjacent surface. The non-planar surface 322 can include, but is not limited to, any non-planar shape, such as an arcuate shape, angular shape, irregular shape, or other non-planar shape. As shown in the example of FIG. 3, the portion 316 the handle 324 is a lower half of the handle 324. The compliant circuit 314 is disposed in the handle 324, on the non-planar surface 322. For instance, the compliant circuit 314 can stretch and conform to the non-planar surface 322 according to the material characteristic of the compliant circuit 314. A portion of the compliant circuit 314 or the entire compliant circuit 314 can be disposed along the non-planar surface 322. In some examples, the compliant circuit 314 can include a logic component 312 (e.g., the logic component 212 as shown in FIG. 2 and described herein). The logic component 312 can be electrically coupled to the compliant circuit 314 by various interconnects, such as a surface mount lead, through-hole lead, ball grid array, wire bonding, conductive adhesive, or the like. In a further example, the logic component 312 can be embedded into the compliant circuit 300 as shown in FIG. 4 and described herein.

By conforming the compliant circuit 300 to the non-planar surface 322 (e.g., inner surface of the handle 324) the internal volume within the handle 324 can remain unobstructed by the compliant circuit 314. Accordingly, the compliant circuit 314 can save space within the handle 324, for instance, to accommodate irrigation tubing, pull wires, or the like.

In some examples, the compliant circuit 314 can be sized and shaped to conform with the non-planar surface 322. Adapting the shape of the compliant circuit 314 to fit along the non-planar surface 322 can increase the size of the compliant circuit 314 that will fit into the handle 324. For instance, a flat circuit can be located across a width (e.g., diameter) of the handle 324. The compliant circuit 314 can conform to the non-planar surface 322 (e.g., along the cross sectional perimeter or circumference of the handle 324), which provides for a greater surface area for the conformal circuit 314 to fit within the handle 324. Accordingly, the surface area of the compliant circuit 314 that fits into the handle 324 can be increased when the size and shape is adapted for the non-planar surface 322. Accordingly, the area used for routing signals (e.g., for traces) and for logic components 312 can be increased.

In some examples, at least a portion of the compliant circuit 314 can be attached to the non-planar surface 322. For instance, the compliant circuit 314, or at least a portion thereof, can be bonded to the non-planar surface 322 with an adhesive. The adhesive can include, but is not limited to, a pressure sensitive adhesive, a thermally activated adhesive, a photo activated adhesive, or other adhesive. In another example, at least a portion of the compliant circuit 314 can be insert molded into the non-planar surface 322. In a further example, the compliant circuit 314 can be thermally formed or thermally bonded to the non-planar surface 322.

The catheter 300 is communicatively coupled with the shaft 302 and the electrical cable 318. In the example of FIG. 3, the shaft 302 and the electrical cable 318 are depicted in an exploded view and decoupled from the compliant circuit 314. The shaft 302 can include a plurality of electrical conductors 306 and a plurality of electrical contacts 304 communicatively coupled to one or more of the electrical conductors 306. In the example of FIG. 3, the shaft 302 can include a flexible circuit. The electrical conductors 306 can be traces along the flexible circuit, and the electrical contacts 304 can be solder pads or exposed conductive pads. In some examples, the flexible circuit can be folded or formed into a cylindrical shape. The proximal end of the flexible circuit can transition to a flatter shape for coupling the electrical contacts 304 with the compliant circuit 314. For instance, the electrical contacts 304 of the shaft 302 can be electrically coupled to a connection interface of the compliant circuit 314, such as a first connection interface 308. In some examples, the electrical contacts 304 can be soldered to the first connection interface 308, bonded to the first connection interface with conductive adhesive, or communicatively coupled by other methods. Accordingly, one or more electrical conductors 306 can be communicatively coupled with the compliant circuit 314.

At the proximal end, the handle 324 is communicatively coupled with the electrical cable 318. The electrical cable 318 can include a plurality of electrical conductors 320. The electrical cable 318 can include, or can be electrically coupled to, one or more flexible circuits 326. In the example of FIG. 3, the electrical cable 318 includes three flexible circuits 326. The electrical conductors 320 can be traces of the flexible circuit 326 and can include solder pads or exposed conductive pads. The electrical conductors 320 can be electrically coupled to a connection interface of the compliant circuit 314, such as a second connection interface 310. In some examples, the electrical conductors 320 can be soldered to the second connection interface 310, bonded to the second connection interface 310 with conductive adhesive, or communicatively coupled by other methods. Accordingly, the compliant circuit 314 can communicate signals between the electrical conductors 306 and the ECU 142 or the ablation generator 122. In some examples, connection interfaces 308, 310, electrical contacts 304, flexible circuit, electrical conductors 320, the electrical conductors 306, 320, or the like can be compliant, as shown in the example of FIG. 3. Correspondingly, the electrical conductors 306, 320 can be communicatively coupled to the connection interfaces 308, 310 along the non-planar surface, such as non-planar surface 322.

**FIG. 4** is an example of a cross section of a compliant circuit 400 including at least one embedded logic component 418. The compliant circuit 400 can include at least one dielectric layer 402, a plurality of conductive layers, and at least one semiconductor 410. For instance, the compliant circuit 400 can include a first conductive layer 404, a second conductive layer 406, and a third conductive layer 408. The conductive layers 404, 406, or 408 can include, but are not limited to, a metal foil, conductive ink, conductive polymer (e.g., printable conductive polymer), or other conductive material. The various conductive layers, such as the first conductive layer 404, the second conductive layer 406, the third conductive layer 408, or a combination thereof, can be configured as a circuit. For instance, various combinations of the conductive layers 404, 406, 408 can be electrically connected using one or more conductive vias within the compliant circuit 400.

In the example of FIG. 4, the first conductive layer 404 can be configured as a source conductor, and the second conductive layer 406 can be configured as a drain conductor. The first conductive layer 404 and the second conductive layer 406 can be separated by a semiconductor 410, such as a semiconductive material disposed over the first conductive layer 404 and the second conductive layer 406. The semiconductor 410 can be configured to switch between an electrically conductive material and an electrically insulative material based on the presence or absence of an electrical field. In some examples, the semiconductor can include a p-type or an n-type semiconductor material. The dielectric material 402 can separate a third conductive layer 408 from the first conductive layer 404 and the second conductive layer 406. In some examples, the compliant circuit 400 can include another dielectric layer 412 to electrically isolate the third conductive layer 408 from other conductors within the compliant circuit 400. In the example of FIG. 4, the third conductive layer 408 can be configured as a gate. An electric charge at the gate can switch the semiconductor 410 between a conductive mode and an insulative mode. Accordingly, the logic component 418 can be a transistor, such as a thin-film transistor (TFT) or an organic-thin-film transistor (OTFT). In some examples, one or more semiconductors (e.g., semiconductor 410) or logic components (e.g., logic component 418) can be separated by a bank 420. The bank 420 can electrically isolate various logic components or semiconductors or mitigate charge migration among different materials.

In some examples, the logic component 418 can be compliant. For instance, the logic component 418 can include a material characteristic that can stretch and comply with a non-planar shape. The semiconductor can include, but is not limited to, organic polymers (e.g., Poly (3-hexylthiophene), polydimethylsiozane, Poly (triarylamines) - Poly(bis04butylphenly-N, N-bisphenyl) benzidine), Ion gel (e.g., (Poly (3-hexylthiophene))), tri-block polymer (e.g., poly (styrene-b-methyl methacrylate-b-styrene), conductive polymer (e.g., poly[(4,8-bis-(2-ethylhexyloxy)-benzo(1,2-b:4,5-b')dithiophene)-2,6-diyl-alt-(4-(2-ethylhexanoyl)-thieno[3,4-b]thiophene-)-2-6-diyl)] (PBDTTT-c) p-doped with the dopant tris-[1-(trifluoroethanoyl)-2-(trifluoromethyl)ethane-1,2-dithiolene] (Mo(tfd-COCF3)3), PMMA, polyvinly alcohol, Polydimethylsilozane, Polyacrylate, acrylaet monoers and oligomers, mercapto-esters and tetrahydrofurfuryl methacrylate), pentacene, α-sexithiophene, dihexyl pentathiophene, copper phthalocyanine, copper hexadecafluorophthalocyanine, poly(3-hexylthiophene) or other semiconductive material. Accordingly, the compliant circuit 400, including the logic components 418 therein, can be compliant to stretch and conform to a non-planar surface.

The logic component 418 can be included in, or configured as, a logic circuit or integrated circuit. In various examples, the logic component 418 can include, but is not limited to, an amplifier, multiplex circuit, transistor, diode, switch, analog-to-digital converter, isolator (filter), or other logic element. Accordingly, the logic component 418 can be embedded within the compliant circuit 400. In some examples, the logic component 418 can be embedded within the compliant circuit 400 and other logic components (e.g., a surface mount electronic component) can be attached (e.g., soldered) to the compliant circuit 400. In further examples, a plurality of logic components 418 can be stacked within the various layers (e.g., conductive and dielectric layers) of the compliant circuit 400. For instance, one or more logic components 418 can be located within inner layers or outer layers of the compliant circuit 400. Accordingly, the logic component 418 can be located above, below, or in between other logic components. By locating one or more logic components 418 within the inner layers of the compliant circuit 400, the overall size (e.g., surface area) of the compliant circuit 400 can be reduced as not all of the logic components need to be located on the outer layers of the compliant circuit 400.

In a further example, embedding the logic component 418 within the compliant circuit 400 can occupy less of the internal volume of the handle (e.g., handle 124, 324) than integrated circuit components attached to the exterior layers of the compliant circuit 400. For instance, logic components, such as surface mount integrated circuits, often can include connection interfaces (e.g., solder connections), overmolds, substrates, leads, and other components that sometimes facilitate a particular application of the logic component. By embedding the logic component 418 in the compliant circuit 400, the size of the logic component can be reduced. For example, the conductive layers 404, 406, 408 of the compliant circuit 400 can be used to electrically couple the logic component 418 to various electrical circuits within the compliant circuit 400, reducing or eliminating the need for leads, connection interfaces, or additional signal routing. In another example, the overmold or cover are unnecessary as the logic component 418 can be supported by a substrate 414. For instance, the substrate 414 can provide mechanical structure for the logic component 418 or for the compliant circuit 400 as a whole. In another example, an encapsulate 416 can protect the logic component 418, such as the semiconductor 410 and conductive layers 404, 406. In some examples, only the desired elements of the logic component 418 can be included in the compliant circuit 400 and unnecessary or duplicative elements can be omitted. Accordingly, the size of the compliant circuit 400 can be reduced or an increased number of logic components 418 can be included in the compliant circuit 400.

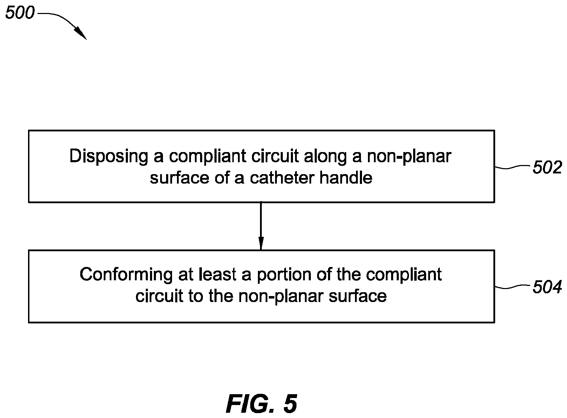

**FIG. 5** is, according to the invention, a method 500 of making a handle of a catheter including a compliant circuit, such as the handle 124, handle 324, compliant circuit 200, compliant circuit 314, or compliant circuit 400 previously described in the examples herein and shown for instance in Figures 1-4. In describing the method 500, reference is made to one or more components, features, functions, and processes previously described herein. Where convenient, reference is made to the components, features, processes and the like with reference numerals. Reference numerals provided are exemplary and are nonexclusive. For instance, features, components, functions, processes, and the like described in the method 500 include, but are not limited to, the corresponding numbered elements provided herein. Other corresponding features described herein (both numbered and unnumbered) as well as their equivalents are also considered.

At 502, a compliant circuit is disposed on a non-planar interior surface of the catheter handle. The non-planar surface can include the non-planar surface 522 as described herein. For instance, the compliant circuit can include any of the compliant circuits 200, 314, or 400 described herein. According to the invention, the compliant circuit includes a dielectric layer having a dielectric material characteristic, such as the dielectric layer 202, 402. The compliant circuit includes a plurality of conductive layers, such as conductive layers 204, 404, 406, 408. For example, each conductive layer can include a conductive material characteristic. In various examples, the conductive layer can include a printable conductive polymer (e.g., a conductive ink), such as a compliant conductive ink having a material characteristic, such as the conductive material characteristic previously described herein. For instance, the conductive layer can be disposed on the dielectric layer using a process including, but not limited to, aerosol jet printing, inkjet printing, screen printing, valve jetting, photolithography & chemical processing, vapor deposition, fused deposition, stereolithography, digital light processing, or the like. For instance, a first layer including at least one dielectric layer or at least one conductive layer can be printed directly on the non-planar surface. A second layer of at least one dielectric layer or at least one conductive layer can be printed on the first layer or on subsequent layers.

In a further example, the conductive layer can include a strain relief feature, such as strain relief feature 206 as described herein. In a further example, the compliant circuit can include a first connection interface electrically coupled to at least one of the conductive layers and a second connection interface electrically coupled to at least one of the conductive layers.

In some examples, the compliant circuit can include one or more logic components, such as one or more of logic components 212, 312, or 418 as shown and described herein. In an example, the logic component can include a semiconductor, for instance, semiconductor 410 shown in FIG. 4 and discussed herein. The logic component can be attached to the compliant circuit or can be embedded within the compliant circuit.

Disposing the compliant circuit on the non-planar surface can include, but is not limited to, insert molding the compliant circuit on to the non-planar surface or bonding the compliant circuit to the non-planar surface. For example, insert molding can include insert molding the catheter handle (e.g., the non-planar surface) to the compliant circuit. In some examples, bonding the compliant circuit to the non-planar surface can include, but is not limited to, attaching the compliant circuit to the non-planar surface with a pressure sensitive adhesive, a thermally activated adhesive, a photo activated adhesive, or other adhesive. In a further example, the compliant circuit can be thermally formed or thermally bonded to the non-planar surface.

At 504, the compliant circuit is conformed to the non-planar surface. The non-planar surface can include any non-planar shape, such as an arcuate shape, angular shape, irregular shape, or other non-planar shape. As previously described herein, the compliant circuit can be conformable along a length and a width of the compliant circuit 200 based on the respective material characteristics of the various layers and elements of the compliant circuit as well as the compliant circuit 200 as a whole. In an example, the compliant circuit 200 can be bent or stretched along a first axis (e.g., X-axis 216), a second axis (e.g., Y-axis 218), or any combination thereof simultaneously based on the respective material characteristics of the compliant circuit 200. Accordingly, the compliant circuit 200 can conform to the non-planar surface. For instance, the compliant circuit can stretch and conform to the non-planar surface to take a shape that corresponds to a shape of the non-planar surface. Stated another way, the compliant circuit can be conformable to the non-planar surface. In some examples, a form can be used to dispose the compliant circuit on the non-planar surface. For instance, the form can include a shape that corresponds with the non-planar shape. The form can be used to stretch and conform the compliant circuit to the non-planar shape *in situ* or before the compliant circuit is disposed on the non-planar shape. For instance, the compliant circuit can be conformed to the non-planar shape and then attached to the non-planar surface of the catheter handle. In a further example, where the non-planar shape is insert molded to the compliant circuit, conforming the compliant circuit to the non-planar surface can include positioning the compliant circuit within a mold (e.g., prior to injection molding the catheter handle).

In further examples, the method 500 can include fabricating the compliant circuit. For instance, fabricating the compliant circuit can include disposing a conductive layer having a conductive material characteristic on a dielectric layer having a dielectric material characteristic. As described previously herein, fabricating the compliant circuit can include printing (e.g., aerosol jet printing) one or more dielectric layers or one or more conductive layers.

Although several examples have been described above with a certain degree of particularity, those skilled in the art could make numerous alterations to the disclosed examples without departing from the scope of the claims. It is intended that all matter contained in the above description or shown in the accompanying drawings shall be interpreted as illustrative only and not limiting. Changes in detail or structure can be made without departing from the present teachings. The scope of the invention is defined by the following claims.

Various examples are described herein of various apparatuses, systems, and methods. Numerous specific details are set forth to provide a thorough understanding of the overall structure, function, manufacture, and use of the examples as described in the specification and illustrated in the accompanying drawings. It will be understood by those skilled in the art, however, that the examples can be practiced without such specific details. In other instances, well-known operations, components, and elements have not been described in detail so as not to obscure the examples described in the specification. Those of ordinary skill in the art will understand that the examples described and illustrated herein are nonlimiting examples, and thus it can be appreciated that the specific structural and functional details disclosed herein can be representative and do not necessarily limit the scope of the examples, the scope of which is defined solely by the appended claims.

Reference throughout the specification to "various examples," "some examples," "one example," "an example," or the like, means that a particular feature, structure, or characteristic described in connection with the example is included in at least one example. Thus, appearances of the phrases "in various examples," "in some examples," "in one example," "in an example," or the like, in places throughout the specification are not necessarily all referring to the same example. Furthermore, the particular features, structures, or characteristics can be combined in any suitable manner in one or more examples. Thus, the particular features, structures, or characteristics illustrated or described in connection with one example can be combined, in whole or in part, with the features structures, or characteristics of one or more other examples without limitation.

It will be appreciated that the terms "proximal" and "distal" can be used throughout the specification with reference to a clinician manipulating one end of an instrument used to treat a patient. The term "proximal" refers to the portion of the instrument closest to the clinician and the term "distal" refers to the portion located furthest from the clinician. It will be further appreciated that for conciseness and clarity, spatial terms such as "vertical," "horizontal," "up," and "down" can be used herein with respect to the illustrated examples. However, surgical instruments can be used in many orientations and positions, and these terms are not intended to be limiting and absolute.

## Claims

1. A handle (124, 324) configured for use with a catheter system, the handle (124, 324) comprising:
a non-planar surface (322) included along an interior of the handle (124, 324);
a distal end configured for coupling with a catheter shaft (128, 302);
a proximal end configured for coupling with an electrical cable (318) for communication with an electronic control unit; and
a compliant circuit (200, 314, 400) disposed on the non-planar surface (322), wherein the compliant circuit (200, 314, 400) is conformed to the non-planar surface (322), and wherein the compliant circuit (200, 314, 400) includes:
a first connection interface (208, 308) located at a distal end of the compliant circuit (200, 314, 400), the first connection interface (208, 308) configured for electrical communication with an electrode or a sensor of the catheter shaft (128, 302), and
a second connection interface (210, 310) located at a proximal end of the compliant circuit (200, 314, 400), the second connection interface (210, 310) configured for electrical communication with the electronic control unit.

2. The handle (124, 324) of claim 1, wherein the compliant circuit (200, 314, 400) includes material configured to stretch and comply with the non-planar surface (322).

3. The handle of claim 1 or 2, wherein the compliant circuit (200, 314, 400) includes at least one dielectric layer (202, 402) and at least one conductive layer (204, 404, 406), wherein at least one of the dielectric layers (202, 402) and at least one of the conductive layers (204, 404, 406) each includes a respective material configured to stretch and comply with the non-planar surface (322).

4. The handle (124, 324) of claim 3, wherein the conductive layer (204, 404, 406) includes a printable conductive polymer.

5. The handle (124, 324) of any one of claims 1to 4, further comprising a logic component embedded within the compliant circuit (200, 314, 400).

6. The handle (124, 324) of claim 5, wherein the logic component is configured as one of an analog-to-digital converter, a multiplexer, a filter, an amplifier, or a combination thereof.

7. The handle (124, 324) of claim 5, wherein the logic component includes a semiconductor comprising a p-type material, an n-type material, or both.

8. The handle (124, 324) of any one of claims 1 to 7, wherein the compliant circuit (200, 314, 400) is insert molded to the non-planar surface (322).

9. A catheter (114, 300) comprising:
a catheter shaft (128, 302) including a proximal end and a distal end having at least one electrode (134, 144) or sensor;
an electrical conductor (156, 306) coupled to the catheter shaft (128, 302), the electrical conductor (156, 306) communicatively coupled to the electrode (134, 144) or the sensor at the distal end of the catheter shaft (128, 302);
the handle (124, 324) of any one of claims 1 to 8.

10. A method of making a catheter handle (124, 324) comprising:
disposing a compliant circuit (200, 314, 400) on a non-planar interior surface (322) of the
catheter handle (124, 324), wherein the compliant circuit (200, 314, 400) includes:
a dielectric layer (202, 402) having a dielectric material configured to stretch and comply with the non-planar surface (322),
a plurality of conductive layers (204, 404, 406), each having a conductive material configured to stretch and comply with the non-planar surface (322),
a first connection interface (208, 308) electrically coupled to at least one of the conductive layers (204, 404, 406), and
a second connection interface (210, 310) electrically coupled to at least one of the conductive layers (204, 404, 406); and
conforming at least a portion of the compliant circuit (200, 314, 400) to the non-planar surface (322).

11. The method of claim 10, wherein disposing the compliant circuit (200, 314, 400) along the non-planar surface (322) includes printing a first layer including at least one dielectric layer (202, 204) or at least one conductive layer (204, 404, 406) directly on the non-planar surface (322), and printing a second layer of at least one dielectric layer (202, 402) or at least one conductive layer (204, 404, 406) on the first layer.

12. The method of claim 10, wherein the compliant circuit (200, 314, 400) is conformed to a shape of the non-planar surface (322) and then disposed and attached to the non-planar surface (322).

13. The method of claim 10, wherein conforming the compliant circuit (200, 314, 400) to the non-planar surface (322) includes positioning the compliant circuit (200, 314, 400) within a mold, and wherein disposing the compliant circuit (200, 314, 400) along the non-planar surface includes insert molding the non-planar surface (322) of the catheter handle (124, 324) to the compliant circuit (200, 314, 400).

14. The method of any one of claims 10 to 13, further comprising fabricating the compliant circuit (200, 314, 400), wherein fabricating the compliant circuit (200, 314, 400) includes disposing the conductive layer on the dielectric layer (202, 402).

## Patentansprüche

1. Handgriff (124, 324), der ausgebildet ist zur Verwendung mit einem Kathetersystem, wobei der Handgriff (124, 324) enthält:
eine nicht-ebene Oberfläche (322), die entlang einer Innenfläche des Handgriffs (124, 324) enthalten ist;
ein entferntes Ende, das ausgebildet ist zum Koppeln mit einem Katheterschaft (128, 302);
ein nahes Ende, das ausgebildet ist zum Koppeln mit einem elektrischen Kabel (318) zur Verbindung mit einer elektronischen Steuereinheit; und
eine nachgebende Schaltung (200, 314, 400), die auf der nicht-ebenen Oberfläche (322) angeordnet ist, wobei die nachgebende Schaltung (200, 314, 400) an die nicht-ebene Oberfläche (322) angepasst ist, und wobei die nachgebende Schaltung (200, 314, 400) aufweist:
eine erste Verbindungsschnittstelle (208, 308), die sich an einem entfernten Ende der nachgebenden Schaltung (200, 314, 400) befindet, wobei die erste Verbindungsschnittstelle (208, 308) ausgebildet ist für eine elektrische Verbindung mit einer Elektrode oder einem Sensor des Katheterschafts (128, 302), und
eine zweite Verbindungsschnittstelle (210, 310), die sich an einem nahen Ende der nachgebenden Schaltung (200, 314, 400) befindet, wobei die zweite Verbindungsschnittstelle (210, 310) ausgebildet ist für eine elektrische Verbindung mit der elektrischen Steuereinheit.

2. Handgriff (124, 324) nach Anspruch 1, wobei die nachgebende Schaltung (200, 314, 400) ein Material aufweist, das ausgebildet ist, dass es sich dehnt und mit der nicht-ebenen Oberfläche (322) übereinstimmt.

3. Handgriff nach Anspruch 1 oder 2, wobei die nachgebende Schaltung (200, 314, 400) wenigstens eine dielektrische Schicht (202, 402) und wenigstens eine leitfähige Schicht (204, 404, 406) aufweist, wobei wenigstens eine aus den dielektrischen Schichten (202, 402) und wenigstens eine aus den leitfähigen Schichten (204, 404, 406) jeweils ein entsprechendes Material aufweist, das ausgebildet ist, dass es sich dehnt und mit der nicht-ebenen Oberfläche (322) übereinstimmt.

4. Handgriff (124, 324) nach Anspruch 3, wobei die leitfähige Schicht (204, 404, 406) ein druckbares leitfähiges Polymer aufweist.

5. Handgriff (124, 324) nach einem der Ansprüche 1 bis 4, ferner enthaltend ein logisches Bauteil, das in die nachgebende Schaltung (200, 314, 400) eingebettet ist.

6. Handgriff (124, 324) nach Anspruch 5, wobei das logische Bauteil ausgebildet ist als eines aus einem Analog-zu-Digital-Wandler, einem Multiplexer, einem Filter, einem Verstärker oder einer Kombination daraus.

7. Handgriff (124, 324) nach Anspruch 5, wobei das logische Bauteil einen Halbleiter aufweist, der ein p-Typ-Material, ein n-Typ-Material oder beides enthält.

8. Handgriff (124, 324) nach einem der Ansprüche 1 bis 7, wobei die nachgebende Schaltung (200, 314, 400) in die nicht-ebene Oberfläche (322) Insert-geformt ist.

9. Katheter (114, 300), enthaltend:
einen Katheterschaft (128, 302), der ein nahes Ende und ein entferntes Ende mit wenigstens einer Elektrode (134, 144) oder wenigstens einen Sensor aufweist;
einen elektrischen Leiter (156, 306), der mit dem Katheterschaft (128, 302) gekoppelt ist, wobei der elektrische Leiter (156, 306) verbindungsmäßig mit der Elektrode (134, 144) oder dem Sensor an dem entfernten Ende des Katheterschafts (128, 302) gekoppelt ist;
den Handgriff (124, 324) nach einem der Ansprüche 1 bis 8.

10. Verfahren zum Herstellen eines Katheterhandgriffs (124, 324), enthaltend:
Anordnen einer nachgebenden Schaltung (200, 314, 400) auf einer nicht-ebenen Innenfläche (322) des Katheterhandgriffs (124, 324), wobei die nachgebende Schaltung (200, 314, 400) aufweist:
eine dielektrische Schicht (202, 402), die ein dielektrisches Material aufweist, das ausgebildet ist, dass es sich dehnt und mit der nicht-ebenen Oberfläche (322) übereinstimmt,
eine Vielzahl von leitfähigen Schichten (204, 404, 406), von denen jede ein leitfähiges Material aufweist, das ausgebildet ist, dass es sich dehnt und mit der nicht-ebenen Oberfläche (322) übereinstimmt,
eine erste Verbindungsschnittstelle (208, 308), die elektrisch mit wenigstens einer aus den leitfähigen Schichten (204, 404, 406) gekoppelt ist, und
eine zweite Verbindungsschnittstelle (210, 310), die elektrisch mit wenigstens einer aus den leitfähigen Schichten (204, 404, 406) gekoppelt ist; und
Anpassen wenigstens eines Bereichs der nachgebenden Schaltung (200, 314, 400) an die nicht-ebene Oberfläche (322).

11. Verfahren nach Anspruch 10, wobei Anordnen der nachgebenden Schaltung (200, 314, 400) entlang der nicht-ebenen Oberfläche (322) Drucken einer ersten Schicht, die wenigstens eine dielektrische Schicht (202, 402) oder wenigstens eine leitfähige Schicht (204, 404, 406) aufweist, direkt auf die nicht-ebene Oberfläche (322) und Drucken einer zweiten Schicht aus wenigstens einer dielektrischen Schicht (202, 402) oder wenigstens einer leitfähigen Schicht (204, 404, 406) auf die erste Schicht enthält.

12. Verfahren nach Anspruch 10, wobei die nachgebende Schaltung (200, 314, 400) an eine Form der nicht-ebenen Oberfläche (322) angepasst wird und dann auf der nicht-ebenen Oberfläche (322) angeordnet und an der nicht-ebenen Oberfläche (322) befestigt wird.

13. Verfahren nach Anspruch 10, wobei Anpassen der nachgebenden Schaltung (200; 314; 400) an die nicht-ebene Oberfläche (322) Positionieren der nachgebenden Schaltung (200, 314, 400) im Inneren einer Form enthält, und wobei Anordnen der nachgebenden Schaltung (200, 314, 400) entlang der nicht-ebenen Oberfläche (322) Insert-Formen der nicht-ebenen Oberfläche (322) des Katheterhandgriffs (124, 324) an die nachgebende Schaltung (200, 314, 400) enthält.

14. Verfahren nach einem der Ansprüche 10 bis 13, ferner enthaltend Herstellen der nachgebenden Schaltung (200, 314, 400), wobei Herstellen der nachgebenden Schaltung (200, 314, 400) Anordnen der leitfähigen Schicht auf der dielektrischen Schicht (202, 404) enthält.

## Revendications

1. Poignée (124, 324) configurée pour être utilisée avec un système de cathéter, la poignée (124, 324) comprenant :
une surface non plane (322) incluse le long d'un intérieur de la poignée (124, 324) ;
une extrémité distale configurée pour le couplage avec une tige de cathéter (128, 302) ;
une extrémité proximale configurée pour le couplage avec un câble électrique (318) pour la communication avec une unité de commande électronique ; et
un circuit souple (200, 314, 400) disposé sur la surface non plane (322), où le circuit souple (200, 314, 400) est conformé à la surface non plane (322), et où le circuit souple (200, 314, 400) comprend :
une première interface de connexion (208, 308) située à une extrémité distale du circuit souple (200, 314, 400), la première interface de connexion (208, 308) étant configurée pour une communication électrique avec une électrode ou un capteur de la tige de cathéter (128, 302), et
une deuxième interface de connexion (210, 310) située à une extrémité proximale du circuit souple (200, 314, 400), la deuxième interface de connexion (210, 310) étant configurée pour une communication électrique avec l'unité de commande électronique.

2. Poignée (124, 324) selon la revendication 1, dans laquelle le circuit souple (200, 314, 400) comprend un matériau configuré pour s'étirer et se conformer à la surface non plane (322).

3. Poignée selon la revendication 1 ou 2, dans laquelle le circuit souple (200, 314, 400) comprend au moins une couche diélectrique (202, 402) et au moins une couche conductrice (204, 404, 406), où au moins une des couches diélectriques (202, 402) et au moins une des couches conductrices (204, 404, 406) comprennent chacune un matériau respectif configuré pour s'étirer et se conformer à la surface non plane (322).

4. Poignée (124, 324) selon la revendication 3, dans laquelle la couche conductrice (204, 404, 406) comprend un polymère conducteur imprimable.

5. Poignée (124, 324) selon l'une quelconque des revendications 1 à 4, comprenant en outre un composant logique intégré dans le circuit souple (200, 314, 400).

6. Poignée (124, 324) selon la revendication 5, dans laquelle le composant logique est configuré comme un convertisseur analogique-numérique, un multiplexeur, un filtre, un amplificateur ou une combinaison de ceux-ci.

7. Poignée (124, 324) selon la revendication 5, dans laquelle le composant logique comprend un semi-conducteur comprenant un matériau de type p, un matériau de type n, ou les deux.

8. Poignée (124, 324) selon l'une quelconque des revendications 1 à 7, dans laquelle le circuit souple (200, 314, 400) est moulé par insertion sur la surface non plane (322).

9. Cathéter (114, 300) comprenant :
une tige de cathéter (128, 302) comprenant une extrémité proximale et une extrémité distale ayant au moins une électrode (134, 144) ou un capteur ;
un conducteur électrique (156, 306) couplé à la tige de cathéter (128, 302), le conducteur électrique (156, 306) étant couplé de manière communicative à l'électrode (134, 144) ou au capteur à l'extrémité distale de la tige de cathéter (128, 302) ;
la poignée (124, 324) selon l'une quelconque des revendications 1 à 8.

10. Procédé de fabrication d'une poignée de cathéter (124, 324) comprenant les étapes consistant à :
disposer un circuit souple (200, 314, 400) sur une surface intérieure non plane (322) de la poignée de cathéter (124, 324), dans lequel le circuit souple (200, 314, 400) comprend :
une couche diélectrique (202, 402) ayant un matériau diélectrique configuré pour s'étirer et se conformer à la surface non plane (322),
une pluralité de couches conductrices (204, 404, 406), chacune ayant un matériau conducteur configuré pour s'étirer et se conformer à la surface non plane (322),
une première interface de connexion (208, 308) couplée électriquement à au moins une des couches conductrices (204, 404, 406), et
une deuxième interface de connexion (210, 310) couplée électriquement à au moins l'une des couches conductrices (204, 404, 406) ; et
conformer au moins une partie du circuit souple (200, 314, 400) à la surface non plane (322).

11. Procédé selon la revendication 10, dans lequel la disposition du circuit souple (200, 314, 400) le long de la surface non plane (322) comprend l'impression d'une première couche comprenant au moins une couche diélectrique (202, 204) ou au moins une couche conductrice (204, 404, 406) directement sur la surface non plane (322), et l'impression d'une deuxième couche d'au moins une couche diélectrique (202, 402) ou d'au moins une couche conductrice (204, 404, 406) sur la première couche.

12. Procédé selon la revendication 10, dans lequel le circuit souple (200, 314, 400) est conformé à une forme de la surface non plane (322), puis disposé et fixé à la surface non plane (322).

13. Procédé selon la revendication 10, dans lequel la conformation du circuit souple (200, 314, 400) à la surface non plane (322) comprend le positionnement du circuit souple (200, 314, 400) à l'intérieur d'un moule, et où la disposition du circuit souple (200, 314, 400) le long de la surface non plane comprend le moulage par insertion de la surface non plane (322) de la poignée de cathéter (124, 324) au circuit souple (200, 314, 400).

14. Procédé selon l'une quelconque des revendications 10 à 13, comprenant en outre la fabrication du circuit souple (200, 314, 400), où la fabrication du circuit souple (200, 314, 400) comprend la disposition de la couche conductrice sur la couche diélectrique (202, 402).
